# EUROPEAN PATENT SPECIFICATION

(11) **EP 0 789 754 B1**
(45) Date of publication and mention of the grant of the patent: **12.07.2000**
(21) Application number: 95935551.2
(22) Date of filing: 06.11.1995
(51) Int. Cl.: C12N 1/20, C07K 14/205, C12P 21/00, A61K 39/106

(54) **H. PYLORI FERMENTATION PROCESS**
H. PYLORI FERMENTATION VERFAHREN
PROCEDE DE FERMENTATION DE H. PYLORI

(30) Priority: 04.11.1994 GB 9422331
(43) Date of publication of application: 20.08.1997
(60) Divisional application: 99203653.3
(73) Proprietor: Chiron S.p.A., 53100 Siena (IT)
(72) Inventor: OLIVIERI, Roberto, I-53100 Costalpino (IT); RAPPUOLI, Rino, I-53010 Monteriggioni (IT); TELFORD, John, Laird, I-53010 Monteriggioni (IT)
(74) Representative: Hallybone, Huw George
(86) International application number: IB9501007
(87) International publication number: WO9614393

(56) References cited:
- EP-A- 0 540 897
- WO-A-93/16723
- WO-A-95/22988
- SIXTH INTERNATIONAL SYMPOSIUM ON HELICOBACTER PYLORI AND ITS DISEASES 16-17 SEPTEMBER 1993; EUROPEAN JOURNAL OF GASTROENTEROLOGY & HEPATOLOGY, vol. 6, no. supp.1, 1994 pages S23-S27, S. KAMIYA ET AL. 'Characteristics of vaculating toxin produced by Helicobacter pylori.'
- JOURNAL OF BIOLOGICAL CHEMISTRY, vol. 267, no. 15, 25 May 1992 pages 10570-10575, T.L. COVER ET AL. 'Purification and Characterization of the vaculating toxin from helicobacter pylori'
- MICROBIOLOGY, vol. 140, no. 9, September 1994 pages 2179-2180, G. SCHAFFER ET AL. 'Glucose metabolism by Helicobacter pylori'
- HELICOBACTER PYLORI INFECTION: PROCEEDINGS OF AN INTERDISCIPLINARY MEETING, 18-19 JUNE 1993: EUROPEAN JOURNAL OF GASTROENTEROLOGY & HEPATOLOGY, vol. 5, no. suppl 2, 1993 pages S76-S78, R. RAPPUOLI ET AL. 'Development of a vaccine against Helicobacter pylori: a short over view'
- FEMS MICROBIOLOGY LETTERS, vol. 124, no. 1, 15 November 1994 pages 55-59, A. MARCHINI ET AL. 'Optimized conditions for the fermentation of Helicobacter pylori and production of vacuolating cytotoxin'
- ARCHIVES OF MICROBIOLOGY, vol. 164, no. 4, 1995 pages 290-293, A. MARCHINI ET AL. 'Cyclodextrins for gowth of Helicobacter pylori and production of vacuolating cytotoxin.'
- MICROBIOLOGY AND IMMUNOLOGY, vol. 38, no. 11, 1994 pages 897-900, M.G. MORSHED ET AL. 'Growth medium containing cyclodextrin and low concentration of horse serum for cultivation of Helicobacter pylori.'
- JOURNAL OF CLINICAL MICROBIOLOGY 33 (10). 1995. 2798-2800. ISSN: 0095-1137, ROOSENDAAL R ET AL 'Recovery of Helicobacter pylori from gastric biopsy specimens is not dependent on the transport medium used.'

## Description

The present invention relates to a new method for the growth of *Helicobacter pylori* and purification of the cytotoxin produced by H. *pylori.* In particular the present application relates to a novel medium for the fermentation of H. *pylori.*

*H. pylori* is a curved gram negative microaerobic bacterium isolated approximately 10 years ago which is associated with type B gastritis in humans. H. *pylori* colonises the human gastric mucosa and establishes a chronic infection that may result in gastric and duodenal ulcers (Blazer, 1990, Journal of Infectious diseases, 161, 629-633) and can be a risk factor in the development of gastric carcimona (Parsonnet et al, 1991, New England Journal of Medicine, 325, 1127-1131). The discovery of the association between H. *pylori* and gastric illness has altered the therapeutic approach to such diseases from the treatment of symptoms with anti-acid drugs to the eradication of the bacterial infection using antibiotics (Marshall, 1993, Gastroenterol. Clin. Northam. 22, 183-198).

In the long term, the infection and diseases thereby occasioned could be prevented and treated by vaccination. The use of Vac A to produce protective antibodies against *H. pylori* infection is described in International patent application WO93/16723 and by Rappuoli *et al,* 1993 (Eur. J. Gastroenterol. and Hepatol. 5, supplement 2, S76-S78). Currently, several factors involved in bacterial adhesion, colonisation and virulence have been identified and are the subjects of investigations into their suitability as vaccine components. One of the most interesting factors is the vacuolating cytotoxin (Vac A) that causes massive vacuolisation in several mammalian cell lines (Luenk, 1991, Rev. Infect. Dis. 13 (supplement 8), S683-S689) and ulceration in mice (Telford *et al,* J. Exp. Med. 179, 1653-1658). The purified cytotoxin is a protein of 87 to 94 KD (Telford *et al, op.* cit.; Cover and Blazer, 1992, J. Biol. Chem. 267, 10570-10575) which may be purified in very small quantities from bacterial culture supernatants. Kamiya *et al,* 1994, (Eur. J. Gastroenterol. and Hepatol. 6, supplement 1, S23-S27) discusses the preparation and partial characterisation of Vac A from *H. pylori* grown in Brucella broth/foetal bovine serum medium. In order to gain an understanding of the mechanism of action of Vac A as well as studying its serology, its role in disease and its utility as a vaccinating antigen large quantities of the protein will be required. Such quantities cannot be obtained using the current methods of culture of H. *pylori* and cytotoxin purification.

Laboratory growth of H. *pylori* is difficult and normally requires complex media containing sera, blood or blood derivatives (Shahamat et *al,* 1991, J. Clin. Microbiol. 29, 2838-2837; Buck & Smith, 1987, J. Clin. Microbiol. 25, 597-599; Morgan *et al*, 1987, J. Clin, Microbiol. 25, 2123-2125). These media interfere with the purification procedures used to isolate the cytotoxin.

Recently, it has been shown that the addition of cyclodextrins to the growth medium can substitute the above-identified additives and sustain the growth of a H. *pylori* (Olivieri *et al*, 1993, J. Clin. Microbiol. 31, 160-162; EP-A-0 540 897). However, growth of the organism remains relatively slow.

Very little is known about the essential growth factors needed by H. *pylori* and about its metabolism in general. For example, although early studies based on acid formation from sugars found no evidence of saccharide fermentation pathways, more recent evidence indicates that H. *pylori* is capable of catabolising saccharides such as glucose. The presence of the pentose phosphate pathway and glucokinase activity have recently been demonstrated (Mendz & Hazzell 1991, FEMS Microbiol. Lett 84, 331-336; Mendz & Hazzell, 1993, Arch. Biochem. Biophys. 300, 522-525) and glucose utilisation has been demonstrated (Mendz *et al,* 1993, J. Gen. Microbiol.139, 3023-3028). However, the extent to which *H. pylori* is capable of metabolising glucose is presently unknown (see Microbiology 140, (1994), 2179-2180).

Although it has been demonstrated that H. *pylori* is capable of utilising glucose, to date it has not been determined whether glucose is a preferred substrate for this organism. Moreover, the effects of glucose utilisation on H. *pylori* remain unknown and it is not clear whether glucose utilisation will affect the growth of the bacterium in a positive or negative manner.

We have now shown that glucose feeding to *H. pylori* cultures results in a substantial improvement in the growth rate of the organism and increases the available biomass at the end of the fermentation process. Moreover, production of the vacuolating cytotoxin is improved. According to a first aspect of the present invention, therefore, there is provided a method for culturing *H. pylori* to produce a vacuolating cytotoxin wherein *H. pylori* is cultured in a medium comprising more than 1 gram per litre of glucose.

Preferably, the glucose is D-glucose. It has been shown that use of glucose in the culture medium results in an increase in the optical density of the medium, which is indicative of the number of *H. pylori* cells present, of almost an order of magnitude. Moreover, production of vacuolating cytotoxin is quadrupled.

Advantageously, the growth medium which is supplemented with glucose is brucella broth, a complex medium composed of tryptone (10 grams per litre), peptamin (10 grams per litre), glucose (1 gram per litre) yeast extract (2 grams per litre) sodium chloride (5 grams per litre) and sodium bisulphite (0.1 gram per litre). The medium may be supplemented with blood derivatives as in the prior art, but advantageously is supplemented with cyclodextrins as previously described (Olivieri *et al*, Op. Cit.) Preferably, about 2 grams of cyclodextrins are used per litre of brucella broth.

The glucose may be added as a single shot addition at the commencement of the fermentation process. Preferably, however, the glucose is added by multiple shot or continuous feeding during the fermentation in a fed batch process. The quantity of glucose fed to the culture should be sufficient to maintain a level of glucose in the medium which is preferably between 2 and 6 grams per litre. Preferably, the concentration of glucose is maintained at or above this level throughout the period of the culture, which is advantageously between 36 and 96 hours.

According to a second aspect of the present invention, there is provided a method for producing *H. pylori* vacuolating cytotoxin comprising culturing *H. pylori* in a medium supplemented with glucose according to the first aspect of the invention.

The vacuolating cytotoxin may be isolated from the bacterial culture medium by any suitable method known in the art. Preferably, however, the vacuolating cytotoxin is isolated by a method which comprises adsorbing the proteins on a cellulose sulphate matrix and subsequently eluting them using a gradient of salt concentrations.

It has been found that the methods previously described in the art, which use adsorbtion onto phenyl sepharose to isolate the vacuolating cytotoxin, are unsatisfactory and give very poor yields, of the order of 0.5%. In contrast, binding to cellulose sulphate is essentially quantitative and yields of 15 - 20% are possible.

The invention further provides a method for the purification of *H. pylori* vacuolating cytotoxin which comprises the steps of:
a) treating the supernatant of an *H. pylori* fermentation in order to concentrate the proteins therein;
b) bringing the proteins into suspension in a buffer comprising a salt concentration equivalent to 100mM NaCl;
c) adsorbing the proteins onto a cellulose sulphate column;
d) eluting the bound proteins from the column using a salt gradient equivalent to 0.1 to 1.5 M NaCl in a phosphate buffer at pH6.5;
e) selecting the fraction of the eluate which contains the vacuolating cytotoxin;
f) optionally, concentrating the cytotoxin further and subjecting it to size separation using a controlled pore matrix.

Methods for concentration of proteins as required by step a) of the above method are known in the art. For example, the proteins can simply be precipitated in ammonium sulphate, preferably 50% saturated ammonium sulphate, and subsequently resuspended in a 100mM NaCl, 20mM phosphate pH 6.5 buffer in step b). Alternatively, the proteins may be concentrated by tangentrial filtration and diafiltration (Miniset OMEGA, cut-off 300kDa, Filtron).

Preferably, the cellulose sulphate column is a Matrex™ column. Elution from the column is preferably carried out with a salt gradient of between 0.1 and 1.5M NaCl, in a pH6.5 phosphate buffer. The fractions eluted from the column are advantageously assayed for VacA by western blotting using a VacA-specific antiserum.

VacA may be isolated by pooling the fractions containing VacA and concentrating the protein, for example by precipitation with ammonium sulphate, resuspension in PBS and subsequent size separation. This latter step may be carried out, for example, on a Superose 6 (Pharmacia) column equilibrated in PBS.

Preferably, the method further compromises culturing *H. pylori* in a medium supplemented with glucose, as described in the first aspect of the invention.

The invention also provides a method for vaccinating an organism against *H. pylori* infection which comprises preparing a sample of VacA as described above and administering a composition comprising the VacA thereby prepared, or a fragment or a derivative thereof which retains a *H. pylori* -specific immunogenicity, to the organism.

Vaccines according to the invention may be formulated according to any technique known in the art.

The vaccines according to the invention may either be prophylactic (to prevent infection) or therapeutic (to treat disease after infection).

Such vaccines comprise antigen or antigens, usually in combination with "pharmaceutically acceptable carriers", which include any carrier that does not itself induce the production of antibodies harmful to the individual receiving the composition. Suitable carriers are typically large, slowly metabolized macromolecules such as proteins, polysaccharides, polylactic acids, polyglycolic acids, polymeric amino acids, amino acid copolymers, lipid aggregates (such as oil droplets or liposomes), and inactive virus particles. Such as carriers are well known to those of ordinary skill in the art. Additionally, these carriers may function as further immunostimulating agents ("adjuvants").

Furthermore, the antigen may be conjugated to a bacterial toxoid, such as toxoid from diphtheria, tetanus, cholera, *H. pylori,* etc. pathogens.

The immunogenic compositions (e.g., the antigen, pharmaceutically acceptable carrier, and adjuvant) typically will contain diluents, such as water, saline, glycerol, ethanol, etc. Additionally, auxiliary substances, such as wetting or emulsifying agents, pH buffering substances, and the like, may be present in such vehicles.

Immunogenic compositions used as vaccines comprise an immunologically effective amount of the adjuvant and an antigen, as well as any other of the above-mentioned components, as needed. By "immunologically effective amount", it is meant that the administration of that amount to an individual, either in a single dose or as part of a series, is effective for treatment or prevention. This amount varies depending upon the health and physical condition of the individual to be treated, the taxonomic group of individual to be treated (e.g., nonhuman primate, primate, etc.), the capacity of the individual's immune system to synthesize antibodies, the degree of protection desired, the formulation of the vaccine, the treating doctor's assessment of the medical situation, and other relevant factors. It is expected that the amount will fall in a relatively broad range that can be determined through routine trials.

Dosage treatment may be as single dose schedule or a multiple dose schedule. The vaccine may be administered in conjunction with other immunoregulatory agents.

In a particularly preferred embodiment, the vaccines according to the invention are administered in combination with a mucosal adjuvant, which is advantageously a mutant of a bacterial ADP-ribosylating toxin, as described in our copending UK patent application No. 9326174.1, entitled "Mucosal Adjuvant".

The invention provides the use of VacA prepared according to the first aspect of the invention on the preparation of a composition for use as a vaccine.

The invention will now be described, for the purposes for illustration only, in the following examples, with reference to the Figures, in which:
Figure 1 shows the growth kinetics of *H. pylori* CCUG 17874 in brucella broth comprising 2gl⁻¹ cyclodextrins in a 7 litre bioreactor;
Figure 2 shows the kinetics of an experiment identical to that shown in Figure 1, except that a glucose feed is employed;
Figure 3 shows the concentration of VacA during *H. pylori* growth with (A) and without (B) glucose feed;
Figure 4 shows the elution profile of proteins from a Matrex column (solid line) measured by absorbance at 280mm. The NaCl gradient is indicated by the broken line;
Figure 5 shows the A₂₈₀ profile of elution of a partially purified cytotoxin from a Superose 6 column;
Figure 6 shows a coomassie blue stained SDS gel of purified cytotoxin (A), and an immunoblot of the material shown in A (B2) and a similar preparation showing processed 37kDa and 58kDa products (B1); and
Figure 7 shows the neutralisation of cytotoxin vacuolating activity by antiserum raised against the purified Vac A.
Figure 8 shows an *H. pylori* growth curve and VacA production in a scaled-up (30L fermentor) reaction, where VacA can be found in the supernatant, VacA(s), and associated with the pellet, VacA(p).

### EXAMPLES

### Example 1

### Production of Vac A in a glucose supplemented medium.

### MATERIALS AND METHODS

Bacterila strain. *Helicobacter pylori* CCUG 17874 (type strain, Culture Collection, University of Goteborg) was used in this study.

Media and supplements. Columbia blood agar (CBA) (Difco), supplemented with the following antibiotics (all from Sigma) Cefsulodin 6 mg l⁻¹, Vancomycin 5 mg l⁻¹, was used as solid medium for CFU determination. Brucella Broth (BB) (Difco) supplemented with 2 gl⁻¹ of (2,6 di-o-methyl)-β-cyclodextrin (CD) (Teijin Lim. Tokyo, Japan) and the antibiotics mentioned above, was used as liquid medium.

Perseveration. Frozen aliquots for inocula were prepared and diluted 1:2 with a solution composed of : glycerol 40%, fetal calf serum (HyClone, Logan, Utah) 20% and 0.4% CD. The suspension obtained was distributed in 1.5ml vials and stored at -80°C.

Growth in Liquid Medium Initial cultures were performed in 130ml Erlenmyer flasks containing 30ml of liquid medium. Cultures were inoculated with 1 ml of frozen stocks and incubated at 36°C for 36 hrs with shaking (100 rpm, 2.5 cm throw) in a microaerobic environment. Flasks were placed inside an anarobic jar where BBL Campy Pak envelopes (Becton Dickinson) were used to generate the proper conditions. These cultures were then used to inoculate 500ml flasks containing 150ml of medium and incubated under the same conditions mentioned above. These cultures were used to inoculate the bioreactors.

Culture vessels and growth conditions. Batch fermentations were carried out in 7 litre bioreactors (MBR Bioreactors AG, 8620 Wetzikon, CH) containing 51 of medium. All cultures were grown at 36°C. The pH was not controlled. The dissolved oxygen tension (DOT) was maintained automatically at the pre-set level (5%) by a two step procedure. First, air flow rate was increased from 0.1 up to 0.5 1 l⁻¹ min⁻¹ to satisfy the increasing O₂ demand of the culture. If further increases were necessary, they were obtained by supplying pure O₂ up to a maximum of 0.4 1 l⁻¹ min⁻¹. A constant flow of N₂ and CO₂ was maintained equal to 0.2 1 l⁻¹ and 0.02 1 l⁻¹ min⁻¹ respectively. The agitation speed was maintained at 130 rpm. The agitator shaft was equipped with two Rhuston turbines having a diameter of 7cm and the diameter of the bioreactor was 17cm.

Glucose feed. A 50% glucose solution was used.

Biomass determination. Growth was monitored by CFU determination and by optical density at 590 nm against a water blank (Perkin Elmer 35 spectrophotometer), light path of 1 cm. Purity checks of the samples were made by Gram staining and by subculturing samples on CBA plates which were incubated in a normal atmosphere at 37°C for 24h.

Quantitative analysis if the VacA protein. At determined time points during the fermentation, culture samples were centrifuged (Biofuge A, Heareus) at 8 300 x g for 10 min.

The supernatants were precipitated with trichloroacetic acid and subjected to a 9% SDS-Page using a BioRad Mini Protean II apparatus. Proteins were transferred to nitrocellulose filters (Schleicher & Schuell) and then incubated overnight with polyclonal antisera raised against the VacA protein (Telford *et al*; *Op. Cit.*). After incubation for 2 hours with a horseradish-peroxidase conjugated secondary antibody (Sigma), the immunoreactive bands were visualized by 4-chloro-naphthol staining. For each immunoblot the amount of cytotoxin in culture supernatants was estimated using a calibration curve obtained with known quantities of purified VacA protein. Quantitative estimation was made by ultrascanner densitometry using Image Master Desk Top Scanner (Pharmacia) in 1D reflectance mode.

Glucose assay. Glu-cinet, glucose test (Sclavo S.p.A., Italy) was used.

### RESULTS

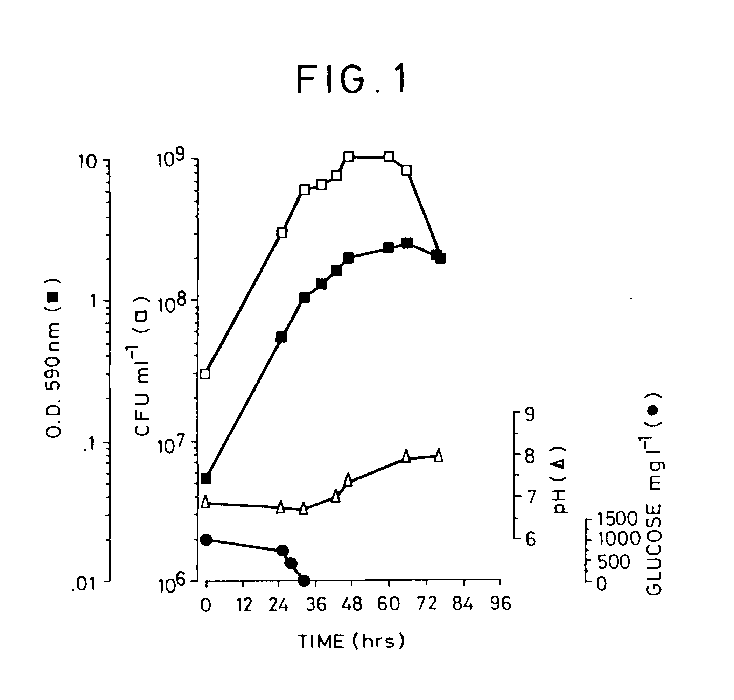

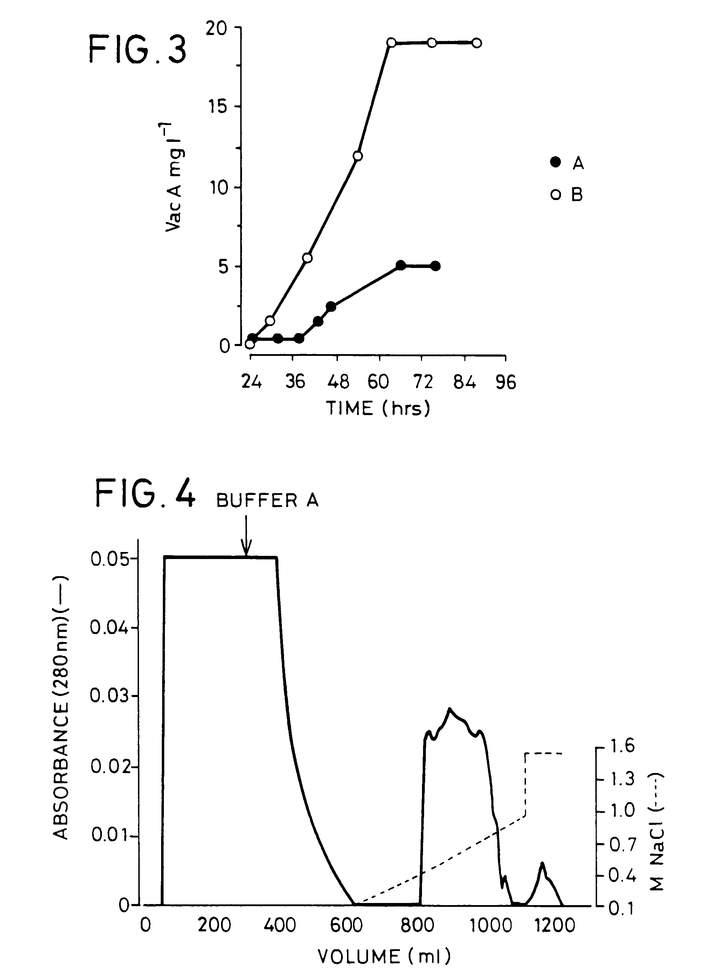

As illustrated in Figure 1, in the absence of glucose feed, the growth of *H. pylori* was exponential as long as glucose was present in the medium and under these conditions the doubling time was estimated to be 7.5 hrs. The stationary phase ensued when the O.D. of the culture reached the value of about 2.5. We also measured the CFU during the various phases of growth (Fig. 1 ). As clearly indicated, the CFU curve correlated well with the O.D. curve as long as glucose was not depleted. In the culture conditions described here, we observed a decrease of pH value of the medium from 6.9 to 6.7 after 33 hours of growth, which then reached the value of 7.4 at the end of the growth phase and a value of 7.9 at the end of the stationary phase. The accumulation of VacA in the broth is reported in Fig. 3(A). It can be seen that the maximum concentration of VacA (5mg l⁻¹) is reached at 65 hrs and remains constant in the stationary phase for at least 10 hrs. From these initial studies were inferred that the amount of VacA produced might be related to the total accumulated cell mass.

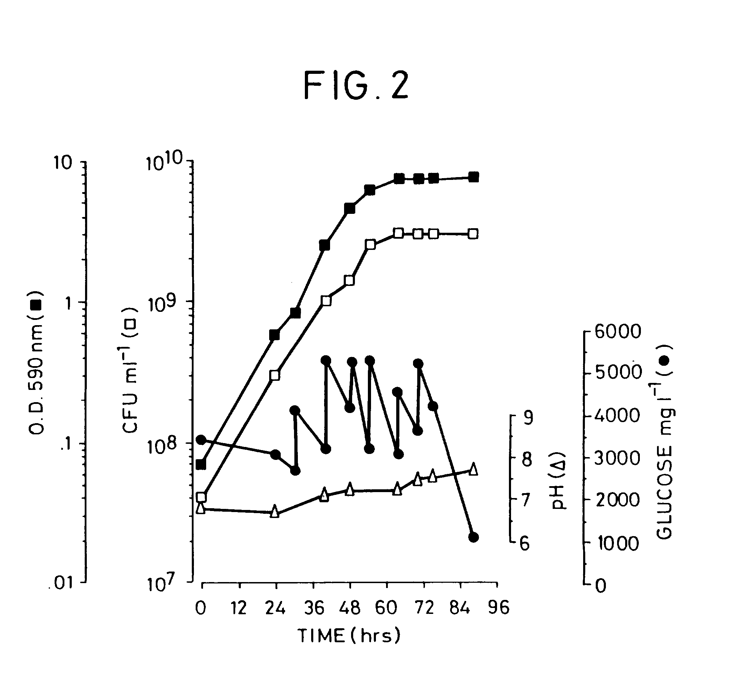

To verify this hypothesis, glucose feeds were used to ensure that this carbon and energy source was always present in the medium. Fig.2 shows that under these conditions, the growth reached a value of 7.70.D. units and a CFU of 3 x 10⁹ml⁻¹. To the contrary of the results obtained when glucose was completely metabolized (Fig.1), in this case the curve of CFU versus time is more related with that obtained using O.D. values. Even the pH values differ significantly in the stationary phase with respect the fermentations without glucose feed (Fig.1). In this type of fermentation, the presence of glucose increased the buffer potency of the medium, due to CO₂ production, and avoided the lysis of the cells. The final pH values were 7.6 against 7.9 of the fermentations without glucose feed. When *H. pylori* was cultured in presence of glucose the concentration of VacA in the medium reached the very promising value of 19mg l⁻¹.

### EXAMPLE 2

### Scale-up

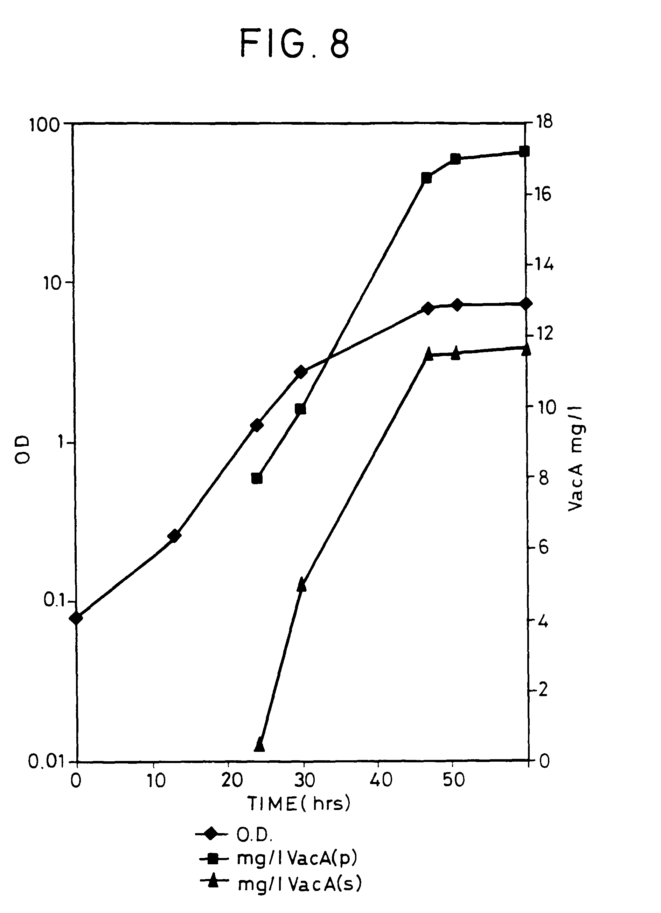

In 30 1 fermentor, containing 15 1 of medium composed of tryptone 10 g/l, Y.E. 5 g/l, NaCl 5 g/l, cyclodextrin 2 g/l, glucose 5 g/l, (this medium is a simplified version of BB) a scaled-up culture of *H. pylori* was performed. Surface aeration was performed maintaining the DOT at 5% with oxygen and agitation. A 5 g/l glucose feed was provided when the OD reaction reached about 3. The doubling time was 5hrs. The pH was not controlled. The growth curve and VacA production obtained are shown in Figure 8, where it is shown that VacA can be found in the supernatant, VacA(s), and associated with the pellet, VacA(p).

### EXAMPLE 3

### Purification of VacA

Biomass from a nominally 51 culture of *Helicobacter pylori* strain CCUG 17874 (Example 1) was removed by centrifugation at 11000 x g for 20 minutes and the supernatant liquid (approx. 41) was brought to 50% saturation by addition of solid ammonium sulphate. The suspension was centrifuged at 11000 x g for 20 minutes and the precipitated proteins were resuspended in 100mM NaCl, 20mM phosphate pH 6.5 (buffer A). The resulting suspension was dialysed extensivley against buffer A.

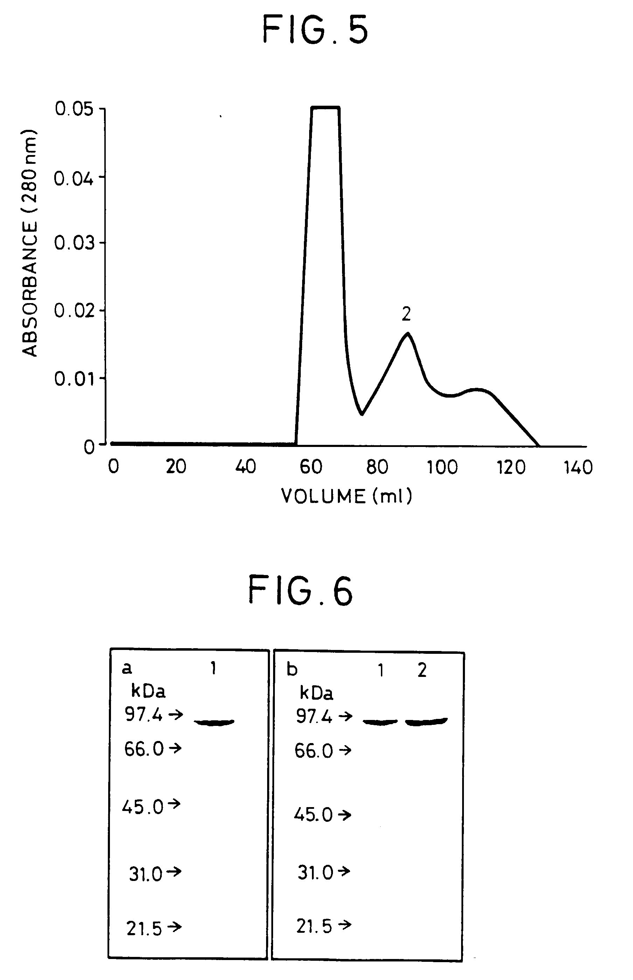

The dialysate was adjusted to a volume of 250ml and applied, at a flow rate of 2.5ml/minute, to a 2.5 x 11cm column (Econo column, Bio Rad) containing Matrex^{TM} (cellulose sulphate, Amicon, Danver, Mass.) equilibrated with buffer A. After extensive washing with buffer A, proteins were eluted from the column using a gradient of NaCl between 0.1-1.5M in 20mM phosphate buffer pH 6.5 and monitored by absorbance at 280nm (Figure 4). The eluate between 0.5 and 0.8M NaCl containing VacA protein was collected and brought to 50% saturation with ammonium sulphate. The suspension was centrifuged at 11000 x g for 20 minutes and the pelleted proteins were resuspended in 3.5ml of PBS. Insoluble material was removed by centrifugation at 85000 x g for 30 minutes and the cleared solution was applied to 16 X 81 mm column packed with Superose 6 (Pharmacia, Uppsala, Sweden). Proteins were eluted with PBS at a flow rate of 14 ml/hour and monitored by absorbance at 280nm (Figure 5).

Fractions from the column corresponding to peak 2 (Figure 5) contained a predominant 94kDa polypeptide when analysed by denaturing polyacrylamide gel electrophoresis and stained with Coomassie blue (Figure 6A). Immunoblotting of these products using specific rabbit anti-VacA antiserum revealed that 94kDa polypeptide was the VacA protein (Figure 6B). Occassionally traces of 37kDa and a 58kDa polypeptides could be detected in immunoblots of purified toxin which correspond to processed molecules as has previously been described by Telford *et al* (J.Exp. Med., 179:1653, 1994).

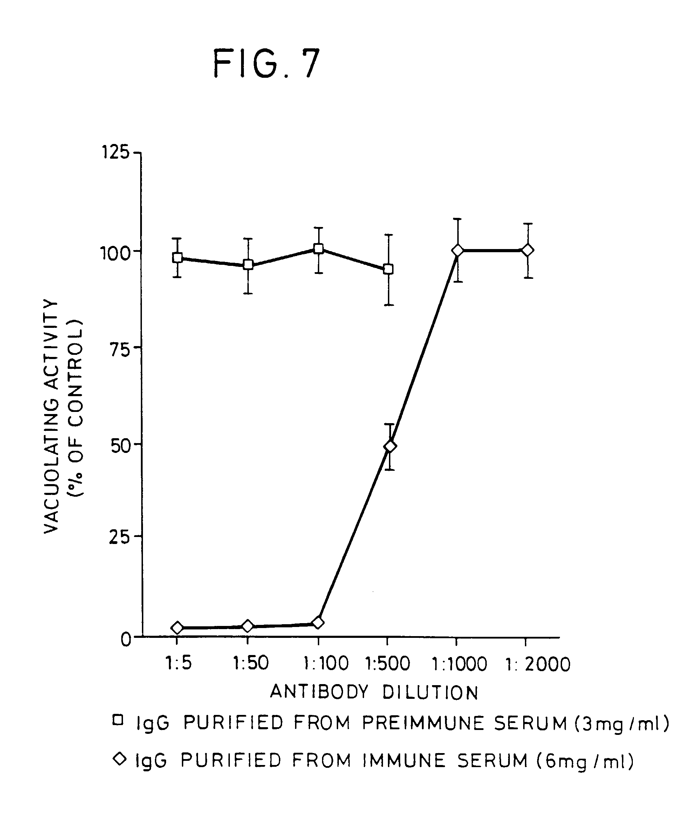

Protein concetration in the purified fractions was measured using the Micro BCA protein assay reagent kit (Pierce, Rockford,Ill) using bovine serum albumin as a standard. Aprroximately lmg of protein /l culture was obtained corresponding to a yeild of 15% of the total toxin. Rabbits were immunized by injecting intradermally 4 doses spaced 7 days apart of 25µg each of purified toxin in 1ml of a solution of 1mg/ml aluminium hydroxide as adjuvant. Immunoglobulins were purified using protein G sepharose and 1tested for their ability to neutralize VacA cytotoxin activity in an *in vitro* vacuolation assay (Papini et al, Mol. Microbiol 7:323, 1993). Serial dilutions of the purified Ig fraction were incubated with 1.85 µg of purified toxin in 100 µg of culture medium before being added to the cells. The antibodies were capable of complete neutralization of cytotoxin activity at a dilution of 1:100 (Figure 7).

### EXAMPLE 4

### Protection of subjects by immunisation with VacA

Mice (6 week old CD1/SPF and BALB/C (Charles River, Calco Italy)) were injected with fresh isolates of *H. pylori* as described in our corresponding U.K. patent application 9419661.5.

In order to asses the possibility of protecting the mice for *H. pylori* infection by immunisation, purified VacA obtained according to the invention was administered orally in combination with an LT mucosal adjuvant on days 1, 14 and 21 with *H. pylori* 100mg of VacA and 100mg LT were administered in each dose.

On days 28, 30 and 32 the immunised mice were challenged with *H. pylori* according to the above infection protocol. On day 42, mice were killed and colonisation of the gastric mucosa by *H. pylori* was assessed. The results are given in Table 1:

**Table 1 -**

| Protection by vaccination with VacA | | |
|---|---|---|
| Treatment | infected | %protection |
| Saline | 5/5 | 0 |
| LT | 3/4 | 25 |
| VacA | 3/4 | 25 |
| VacA + LT | 1/3 | 77 |

## Claims

1. A method for culturing *H. pylori* to produce a vacuolating cytotoxin, wherein *H. pylori is* cultured in a medium comprising more than 1 gl⁻¹ of glucose.

2. A method according to claim 1 wherein the medium is Brucella Broth medium supplemented with glucose and blood products.

3. A method according to claim 1 wherein the medium is Brucella Broth medium supplemented with glucose and a cyclodextrin.

4. A method according to any preceding claim wherein the glucose is supplied by multiple-shot or continuous feeding in a fed batch process.

5. A method according to any preceding claim wherein the glucose concentration of the medium is maintained at between 2 and 6 gl⁻¹ throughout the culture period.

6. A method for producing *H. pylori* vacuolating cytotoxin comprising culturing *H. pylori* in a medium supplemented with glucose as described in any one of claims 1 to 5.

7. A method according to claim 6 further comprising purifying the cytotoxin by adsorbing it onto a cellulose sulphate matrix and subsequently eluting it using a gradient of salt concentrations.

8. A method for the purification of *H. pylori* vacuolating cytotoxin which comprises the steps of:
a) treating the supernatant of an *H. pylori* fermentation cultured according to the method of any one of the preceding claims in order to concentrate the proteins therein;
b) bringing the proteins into suspension in a buffer comprising a salt concentration equivalent to 100mM NaCl;
c) adsorbing the proteins onto a cellulose sulphate column;
d) eluting the bound proteins from the column using a salt gradient equivalent to 0.1 to 1.5 M NaCl in a phosphate buffer at pH6.5;
e) selecting the fraction of the eluate which contains the vacuolating cytotoxin;
f) optionally, concentrating the cytotoxin further and subjecting it to size separation using a controlled pore matrix.

9. A method according to claim 8, wherein step a) comprises precipitation in ammonium sulphate.

10. A method according to claim 8, wherein steps a) and b) comprise tangential filtration and diafiltration of the sample.

11. A method according to claim 7 wherein the cytotoxin purification is performed as described in any one of claims 8 to 10.

12. A process for the manufacture of a medicament for use as a vaccine comprising the steps of:
a) culturing *H. pylori* to produce a vacuolating cytotoxin using a method according to any one of claims 1 to 11, and
b) bringing said vacuolating cytotoxin into association with a suitable pharmaceutical excipient.

## Patentansprüche

1. Verfahren zur Züchtung von H. pylori zur Produktion eines Vakuolenbildendenden Cytotoxins, wobei H. pylori in einem Medium gezüchtet wird, das mehr als 1 gl⁻¹ Glucose umfaßt.

2. Verfahren nach Anspruch 1, wobei das Medium Brucellabrühe-Medium ist, das mit Glucose und Blutprodukten ergänzt ist.

3. Verfahren nach Anspruch 1, wobei das Medium Brucellabrühe-Medium ist, das mit Glucose und einem Cyclodextrin ergänzt ist.

4. Verfahren nach einem der vorangehenden Ansprüche, wobei die Glucose durch Mehrfachzugabe oder kontinuierliche Zugabe in einem Fed batch-Verfahren zugesetzt wird.

5. Verfahren nach einem der vorangehenden Ansprüche, wobei die Glucosekonzentration des Mediums während des gesamten Züchtungszeitraumes zwischen 2 und 6 gl⁻¹ gehalten wird.

6. Verfahren zur Produktion von Vakuolen-bildendem Cytotoxin von H. pylori, umfassend das Züchten von H. pylori in einem Medium, das mit Glucose ergänzt ist, gemäß der Beschreibung in einem der Ansprüche 1 bis 5.

7. Verfahren nach Anspruch 6, das ferner die Reinigung des Cytotoxins durch Adsorption an eine Cellulosesulfat-Matrix und anschließende Elution unter Verwendung eines Salzkonzentrationsgradienten umfaßt.

8. Verfahren zur Reinigung von Vakuolen-bildendem Cytotoxin von H. pylori umfassend die Schritte:
a) Behandlung des Überstandes einer H. pylori-Fermentation, die gemäß dem Verfahren nach einem der vorangehenden Ansprüche gezüchtet wurde, um die Proteine darin zu konzentrieren;
b) Einbringen der Proteine in eine Suspension in einen Puffer, der eine Salzkonzentration umfaßt, die äquivalent ist zu 100 mM NaCl;
c) Adsorption der Proteine an eine Cellulosesulfat-Säule;
d) Elution der gebundenen Proteine von der Säule unter Verwendung eines Salzgradienten, der äquivalent ist zu 0,1 bis 1,5 M NaCI in einem Phosphatpuffer bei pH 6,5;
e) Auswählen der Fraktion des Eluates, die das Vakuolen-bildende Cytotoxin enthält;
f) gegebenenfalls weitere Konzentration des Cytotoxins und Durchführung einer Größentrennung unter Verwendung einer Matrix mit geregelten Poren.

9. Verfahren nach Anspruch 8, wobei Schritt a) die Ausfällung in Amoniumsulfat umfaßt.

10. Verfahren nach Anspruch 8, wobei die Schritte a) und b) tangentiale Filtration und Diafiltration der Probe umfassen.

11. Verfahren nach Anspruch 7, wobei die Cytotoxinreinigung gemäß der Beschreibung in einem der Ansprüche 8 bis 10 durchgeführt wird.

12. Verfahren zur Herstellung eines Medikamentes zur Verwendung als Impfstoff, umfassend die Schritte:
a) Züchten von H. pylori zur Produktion eines Vakuolen-bildenden Cytotoxins unter Verwendung eines Verfahrens nach einem der Ansprüche 1 bis 11, und
b) Zusammenbringen des Vakuolen-bildenden Cytotoxins mit einem geeigneten pharmazeutischen Excipienten.

## Revendications

1. Procédé de culture de *H. pylori* pour la production d'une cytotoxine vacuolisante, dans lequel *H. pylori* est cultivé dans un milieu comprenant plus de 1 g.l⁻¹ de glucose.

2. Procédé selon la revendication 1, dans lequel le milieu est du bouillon Brucella complété avec du glucose et des produits sanguins.

3. Procédé selon la revendication 1, dans lequel le milieu est du bouillon Brucella complété avec du glucose et une cyclodextrine.

4. Procédé selon l'une quelconque des revendications précédentes, dans lequel le glucose est fourni par alimentation par additions multiples ou continue dans un processus discontinu avec additions.

5. Procédé selon l'une quelconque des revendications précédentes, dans lequel la concentration du glucose dans le milieu est maintenue entre 2 et 6 g.l ⁻¹ pendant toute la durée de la culture.

6. Procédé de production de la cytotoxine vacuolisante de *H. pylori,* comprenant la culture de *H. pylori* dans un milieu complété avec du glucose, comme décrit dans l'une quelconque des revendications 1 à 5.

7. Procédé selon la revendication 6, comprenant en outre la purification de la cytotoxine par adsorption de celle-ci sur une matière de support à base de sulfate de cellulose et ensuite son élution à l'aide d'un gradient de concentrations de sel.

8. Procédé pour la purification de la cytotoxine vacuolisante de *H. pylori,* comprenant les étapes suivantes:
a) traitement du surnageant d'un bouillon de fermentation de *H. pylori* cultivé selon le procédé de l'une quelconque des revendications précédentes, afin de concentrer les protéines contenues dans celui-ci;
b) mise des protéines en suspension dans un tampon comprenant une concentration de sel équivalente à 100 mM de NaCl;
c) adsorption des protéines sur une colonne de sulfate de cellulose;
d) élution des proteines fixées de la colonne à l'aide d'un gradient de sel équivalent de 0,1 à 1,5 M de NaCl dans un tampon phosphate à pH 6,5;
e) sélection de la fraction de l'éluat qui contient la cytotoxine vacuolisante;
f) éventuellement, concentration plus poussée de la cytotoxine et soumission de celle-ci à un fractionnement en fonction de la taille, au moyen d'une matière de support à taille de pores réglée.

9. Procédé selon la revendication 8, dans lequel l'étape a) comprend la précipitation dans du sulfate d'ammonium.

10. Procédé selon la revendication 8, dans lequel les étapes a) et b) comprennent une filtration tangentielle et une diafiltration de l'échantillon.

11. Procédé selon la revendication 7, dans lequel la purification de la cytotoxine est effectuée comme décrit dans l'une quelconque des revendications 8 à 10.

12. Procédé de fabrication d'un médicament destiné à être utilisé comme vaccin, comprenant les étapes suivantes:
a) culture de H. *pylori* pour la production d'une cytotoxine vacuolisante, à l'aide d'un procédé selon l'une quelconque des revendications 1 à 11, et
b) mise de cette cytotoxine vacuolisante en association avec un excipient pharmaceutique approprié.
